# EUROPÄISCHE PATENTSCHRIFT

(11) **EP 3 170 574 B1**
(45) Veröffentlichungstag und Bekanntmachung des Hinweises auf die Patenterteilung: **13.07.2022**
(21) Anmeldenummer: 15195313.0
(22) Anmeldetag: 19.11.2015
(51) Int. Cl.: B21D 28/06, B21D 35/00, B21D 51/54, A61N 1/05

(54) **VERFAHREN ZUR HERSTELLUNG EINER HÜLSE FÜR EINE ELEKTRODE FÜR MEDIZINISCHE ANWENDUNGEN**
METHOD FOR THE MANUFACTURE OF A SLEEVE FOR AN ELECTRODE FOR MEDICAL APPLICATIONS
PROCEDE DE FABRICATION D'UN MANCHON POUR UNE ELECTRODE POUR APPLICATIONS MEDICALES

(43) Veröffentlichungstag der Anmeldung: 24.05.2017
(73) Patentinhaber: Heraeus Deutschland GmbH & Co. KG, 63450 Hanau (DE)
(72) Erfinder: DÖRGE, Thomas, 66538 Neunkirchen (DE); LEITOLD, Christiane, 61200 Wölfersheim (DE); KEITEL, Oliver, 63741 Aschaffenburg (DE); SCHNEIDER, Dominik, 63826 Geiselbach (DE); MUSIOL, Katharina, 63452 Hanau (DE); ROTH, Josef, 63856 Bessenbach (DE); KOCH, Bernd, 42279 Wuppertal (DE); KOCH, Benjamin, 42279 Wuppertal (DE); SCHIEFER, Herwig, 60389 Frankfurt (DE)
(74) Vertreter: Heraeus IP

(56) Entgegenhaltungen:
- EP-A1- 2 875 844
- EP-A2- 1 323 483
- EP-A2- 2 772 280
- JP-A- S5 829 598
- US-A1- 2015 223 713

## Beschreibung

Die Erfindung betrifft Verfahren zur Herstellung einer metallischen Hülse als Ringelektrode für elektrophysiologische und neuromedizinische Anwendungen sowie Verfahren zum Herstellen eines neuromedizinischen Sensors oder einer elektrophysiologischen Ringelektrode unter Anwendung eines solchen Verfahrens zur Herstellung der Hülse.

Im Bereich des Cardiac-Rhythm-Management (CRM) oder im elektrophysiologischen und neuromedizinischen Bereich werden Ringelektroden aus Edelmetall zur Stimulation und/oder zur Erfassung von bioelektrischen Signalen eingesetzt. Typischerweise werden diese Ringelektroden in Form von Hülsen, die als durchgehende Röhrchen aufgebaut sind, durch einen Drehprozess ("swiss machining") hergestellt und müssen aus einem Stab oder ähnlichem unter großem Verlust des Edelmetalls gefertigt werden. Das Verfahren ist darüber hinaus teuer und aufwendig durch die langen notwendigen Bearbeitungszeiten. Es kann mit einem Drehprozess nur in etwa eine Hülse in zwei bis drei Minuten gefertigt werden. Weiterhin ist dieser Prozess limitiert in Bezug auf sehr dünne Wandstärken, die durch einen Drehprozess nicht ohne zusätzlichen Aufwand gefertigt werden können. Zudem nutzen sich die Werkzeuge (insbesondere die Fräsköpfe oder Schneidwerkzeuge) beim Abdrehen des überschüssigen Materials ab und müssen in regelmäßigen Abständen ersetzt werden. Der Drehprozess unterliegt auch höheren Variationen aufgrund von Werkzeugverschleiß. Dadurch unterliegt dieser Prozess auch einer höheren Variation der Bauteilgeometrie.

Aus der US 2015/0223713 A1 ist eine Ringelektrode zur elektrischen Neurostimulation bekannt. Dabei wird eine Hülse in Form eines Trägerrohrs mit unterschiedlichen Außendurchmessern eingesetzt, um einen Chip als Sensor zu tragen und mit elektrischen Leitungen zu verbinden. Der Aufbau der Hülse ist aufwendig zu realisieren und kann nicht alleine durch Drehen gefertigt werden.

Die EP 2 875 844 A1 offenbart eine Ringelektrode zur elektrophysiologischen beziehungsweise neuromedizinischen Stimulation und Messung von elektrischen Signalen im Körper. Eine weitere Ringelektrode ist aus der EP 2 772 280 A2 bekannt. Dort werden Hülsen zum Aufbau der Ringelektrode verwendet, die zwei Enden mit unterschiedlichen Außendurchmessern aufweisen und die über spiralförmig gewundene Verbindungsdrähte elektrisch kontaktiert sind. Durch das Innere der Hülsen wird ein weiteres spiralförmig gewundenes Kabel als Innenwendel durchgeführt, mit dem eine Spitzenelektrode kontaktiert wird, mit der ein elektrischer Impuls als Schrittmacher verabreicht werden kann.

Solche Hülsen werden durch Drehen oder andere spanabhebende Verfahren aus massiven Edelmetallkörpern herausgearbeitet. Hierbei entsteht ein erheblicher Aufwand, um die Hülsen mit der gewünschten Genauigkeit herstellen zu können. Zudem entsteht eine große Menge Edelmetall-Abfälle, die aufwendig wiederaufbereitet werden müssen. Für eine schnelle und kostengünstige Massenfertigung sind derartige Verfahren ungeeignet.

Die EP 1 323 483 A2, die die Basis für den Oberbegriff des Anspruchs 1 bildet, betrifft ein Metallrohr unregelmäßiger Form, das mindestens zwei Innendurchmesser hat, wobei das Metallrohr einen kleinen Durchmesser aufweist, so dass es beispielsweise als Nadel, Injektionsnadel oder Stecker verwendet werden kann. Zur Herstellung wird die Vorform einer Hülse beziehungsweise eines Rohres aus einem Metalldünnblech ausgestanzt. Das ausgestanzte Blechteil wird anschließend zu einem rohrförmigen Körper pressgeformt und das so erhaltene Halb-Rohr am Saum verschweißt. Nachteilig hierbei ist, dass ein aufwändiger Schweißschritt zur Herstellung des dünnen Rohrs erforderlich ist.

Die Aufgabe der Erfindung besteht also darin, die Nachteile des Stands der Technik zu überwinden. Insbesondere soll ein Verfahren zur Herstellung einer Hülse für eine Ringelektrode für elektrophysiologische und neuromedizinische Anwendungen bereitgestellt werden, das einfach, schnell und kostengünstig durchzuführen ist. Das Verfahren soll also kostengünstig zu realisieren sein und nur dabei eine möglichst geringe Menge an Edelmetallabfällen produzieren. Das Verfahren soll zudem schnell durchführbar sein, um in möglichst kurzer Zeit Hülsen mit der geforderten Genauigkeit erzeugen. Aufgrund der geringen Mengen und den unterschiedlich einsetzbaren und dadurch variablen Hülsen soll es bevorzugt möglich sein, das Verfahren durch eine Modifizierung des Aufbaus beziehungsweise der Maschine, mit der das Verfahren durchgeführt wird, leicht und schnell derart umzugestalten, dass die erzeugte Hülsenform variiert beziehungsweise verändert wird. Des Weiteren soll die Gefahr einer Beeinträchtigung der Drähte zur elektrischen Kontaktierung der Ringelektrode und der Hülse bei der anschließenden Weiterverarbeitung möglichst gering sein. Hierfür sollen bereits der Aufbau und die Form der Hülse selbst geeignet sein.

Die Aufgaben der Erfindung werden gelöst durch ein Verfahren zur Herstellung einer Hülse zum Aufbau einer Ringelektrode für elektrophysiologische und neuromedizinische Anwendungen aus einem metallischen Band, wobei das Band und die daraus gefertigte Hülse aus einem bio kompatiblen Metall oder einer biokompatiblen metallischen Verbindung bestehen, dadurch gekennzeichnet, dass sich wiederholende Strukturen in das Band gestanzt werden, wobei die sich wiederholenden Strukturen jeweils zumindest eine Fläche aufweisen, die über wenigstens einen Steg mit wenigstens einem äußeren Streifen verbunden sind, wobei zumindest der wenigstens eine äußere Streifen die sich wiederholenden Strukturen randseitig miteinander verbindet, anschließend mehrere der zumindest einen Flächen der sich wiederholenden Strukturen in mehreren Umformschritten durch Tiefziehen und/oder einer anderen Umformtechnik in Hülsen-Form gebracht werden und die Hülsen-Form anschließend freigestanzt wird, so dass die Hülse einen ersten rohrförmigen Bereich mit einem größeren

Durchmesser aufweist und einen zweiten rohrförmigen Bereich mit einem kleineren Durchmesser aufweist, wobei der erste Bereich mit dem größeren Durchmesser einen größeren Außendurchmesser und Innendurchmesser aufweist als der zweite Bereich mit dem kleineren Durchmesser und die beiden Bereiche einteilig miteinander verbunden sind.

Es kann vorgesehen sein, dass der zweite Bereich mit dem kleineren Durchmesser und die beiden Bereiche derart einteilig miteinander verbunden sind, dass die Hülse ein durchgehendes Röhrchen bildet oder ein einseitig geschlossenes Röhrchen bildet. Es kann also auch vorgesehen sein, dass das Röhrchen auf einer Seite durch eine Deckfläche geschlossen ist.

Dabei kann vorgesehen sein, dass der Außendurchmesser des ersten Bereichs mit dem größeren Außendurchmesser zumindest 0,1 mm größer ist als der Außendurchmesser des zweiten Bereichs mit dem kleineren Außendurchmesser.

Mit der vorliegenden Erfindung wird vorgeschlagen, dass das Band und die daraus gefertigte Hülse aus einem Edelmetall, aus Titan, aus Tantal oder einer metallischen Verbindung daraus oder einer metallischen Verbindung enthaltend zumindest ein Edelmetall als Hauptkomponente oder Titan oder Tantal als Hauptkomponente bestehen.

Diese Materialien sind zum Aufbau von elektrophysiologischen Elektroden im medizinischen Bereich aufgrund ihrer chemischen Beständigkeit besonders gut geeignet.

Unter einer Hauptkomponente einer metallischen Verbindung wird vorliegend verstanden, dass zumindest 50 Gew% der Verbindung aus einem Edelmetall oder einer Mischung von Edelmetallen oder aus Titan oder aus Tantal bestehen. Ferner muss das Metall oder die metallische Verbindung biokompatibel sein, damit keine Vergiftung oder eine andere Beeinträchtigung des Körpers des Patienten auftreten kann. Zudem muss die Verbindung metallisch sein, darf bei Raumtemperatur also weder halbleitend noch isolierend sein. Bevorzugt wird als Material für das metallische Band eine intermetallische Legierung als metallische Verbindung verwendet.

Die fertige Hülse kann beidseitig offen sein oder einseitig geschlossen sein. Die Hülsen-Form kann beispielsweise dadurch freigestanzt werden, dass die Stege direkt an der vorgeformten Hülsen-Form getrennt werden. Zudem kann eine geschlossene Deckfläche der Hülse beziehungsweise der Hülsen-Form durch Freistanzen geöffnet werden.

Für elektrophysiologische und neuromedizinische Anwendungen ist es wichtig, dass das Material, aus dem die Hülsen gefertigt sind, biokompatibel ist. Gleichzeitig sollte das Material der Hülsen chemisch inert beziehungsweise möglichst beständig sein, um eine Veränderung der elektrischen Eigenschaften der Hülse zu vermeiden und um eine Vergiftung oder Beeinträchtigung des umgebenden Gewebes ausschließen zu können. Hierfür sind insbesondere Edelmetalle geeignet, insbesondere Palladium, Platin, Gold sowie Platin- und Goldlegierungen, wie beispielsweise Platin-Rhodium- oder Platin-Iridium-Legierungen.

Bevorzugt sind die zumindest einen Flächen der sich wiederholenden Strukturen bezüglich des ausgestanzten Bands innenliegend und werden besonders bevorzugt seitlich von zwei sich am Rand des Bands über mehrere der sich wiederholenden Strukturen erstreckenden äußeren Streifen flankiert. Ebenfalls kann bevorzugt vorgesehen sein, dass die zumindest einen Flächen der Strukturen über zumindest zwei, besonders bevorzugt über zumindest drei Stege, ganz besonders bevorzugt über vier Stege, mit einem, besonders bevorzugt mit zumindest zwei äußeren Streifen verbunden sind. Durch die genannten Maßnahmen wird die Stabilität des gestanzten Bands erhöht und damit die Weiterverarbeitung erleichtert. Ebenfalls bevorzugt sind die zumindest einen Flächen kreisrund oder derart geformt, dass das Verhältnis zwischen dem größten Querschnitt der Fläche ohne die Stege zu dem kleinsten Querschnitt der Fläche höchstens 3 beträgt, besonders bevorzugt höchstens 2 beträgt, ganz besonders bevorzugt höchstens 1,5 beträgt. Zumindest die Flächen des Bands bestehen aus dem Metall beziehungsweise der metallischen Legierung, bevorzugt besteht das gesamte Band aus dem Metall oder der metallischen Legierung, da hierdurch ein einfaches Prozessieren des Bands möglich ist. Alternativ können der zumindest eine äußere Streifen und/oder der wenigstens eine Steg aus einem Kunststoff oder einem anderen Material bestehen, das mit dem metallischen Band oder der metallischen Legierung verbunden ist, bevorzugt laminiert ist. Dadurch kann noch etwas mehr Edelmetall eingespart werden. Dafür ist die Herstellung des Bands etwas aufwendiger. Bei Stanzen kann bei dieser Ausführung auch der Kunststoffstreifen an dem metallischen Band durch Stanzen geformt werden.

Es kann vorgesehen sein, dass mehrere Flächen nebeneinander in jeder der sich wiederholenden Strukturen im metallischen Band vorgesehen sind, beziehungsweise eingestanzt werden, wobei die nebeneinander angeordneten Flächen über Stege miteinander verbunden sind. Bevorzugt sind dabei die äußeren Flächen über Stege mit seitlichen Streifen verbunden, die sich senkrecht zu den Flächen der sich wiederholenden Strukturen entlang des Bands erstrecken. Hierdurch können mit einer Pressung mehrere Hülsen parallel hergestellt werden. Dies ist insbesondere dann vorteilhaft, wenn die Hülsen in kurzer Zeit und in großer Stückzahl gefertigt werden sollen.

Bei erfindungsgemäßen Verfahren kann vorgesehen sein, dass alle Umformschritte, und bevorzugt auch das Stanzen der sich wiederholenden Strukturen und/oder das Freistanzen der Hülsen-Form, mit einem einzigen Werkzeug erfolgt, bei dem das Werkzeug auf das metallische Band gepresst wird, wobei in dem Werkzeug mehrere Module in einer Reihe hintereinander angeordnet sind und mit jedem Modul ein Umformschritt oder ein Freistanzschritt oder ein Stanz-Umform-Schritt durchgeführt wird.

Hintereinander angeordnet bezieht sich vorliegend auf die Förderrichtung des metallischen Bands, das durch das Werkzeug gefördert und dabei umgeformt wird.

Hierdurch kann das Verfahren mit einem kompakten Werkzeug platzsparend und aufgrund der kurzen Förderwege schnell durchgeführt werden. Zudem kann durch den modularen Aufbau des Werkzeugs, das Werkzeug für eine große Zahl verschiedener Hülsen eingesetzt werden, indem einzelne Module ausgetauscht werden. Dies reduziert die Kosten für die Umrüstung des Werkzeugs und damit für die Hülsen, da die Werkzeuge zum Stanzen und/oder Umformen die meisten Kosten verursachen.

Dabei kann vorgesehen sein, dass nach jedem Stanzen und/oder Umformen das Band zum nächsten Modul weiter transportiert wird, wobei vorzugsweise die Abstände aller Module zueinander in Förderrichtung des metallischen Bands immer gleich groß sind.

Dies verdeutlicht, wie ein besonders bevorzugtes und vorteilhaftes Verfahren zur Massenfertigung der Hülsen durchgeführt werden kann. Mit diesem Verfahren lassen sich in kurzer Zeit große Mengen der Hülsen kostengünstig fertigen.

Des Weiteren kann vorgesehen sein, dass zumindest vier, bevorzugt zumindest fünf, chronologisch separate Umformschritte erfolgen, um die Hülsen-Form zu erzeugen, wobei vorzugsweise das Werkzeug hierzu zumindest vier Module, besonders bevorzugt zumindest fünf Module umfasst.

Hierdurch wird sichergestellt, dass bei jedem Umformschritt beziehungsweise Tiefzieh-Schritt des metallischen Bands, der bevorzugt mit einem Modul durchgeführt wird, das Material des Bands beim Umformen nicht so stark beansprucht wird, dass die Gefahr besteht, dass das Band reißt oder Risse bekommt.

Mit einer Weiterbildung des erfindungsgemäßen Verfahrens wird vorgeschlagen, dass das metallische Band und die daraus gefertigten Hülsen aus Tantal, Titan, Palladium, Platin, Gold oder einer Platin-Iridium-Legierung, einer Legierung aufweisend zumindest zwei der Elemente Tantal, Titan, Palladium, Platin, Gold und Iridium oder einer metallischen Verbindung mit Gold und/oder Platin als Hauptkomponente bestehen.

Diese Materialien sind für elektrophysiologische und neuromedizinische Anwendungen besonders vorteilhaft und lassen sich mit dem erfindungsgemäßen Verfahren gut verarbeiten.

Zur besseren Verarbeitung kann vorgesehen sein, dass das Band vor oder direkt nach dem Einstanzen der sich wiederholenden Strukturen geglüht wird, bevorzugt bei einer Temperatur zwischen 300° C und 1000° C geglüht wird, besonders bevorzugt bei einer Temperatur zwischen 500° C und 950° C geglüht wird, ganz besonders bevorzugt bei einer Temperatur zwischen 500° C und 700° C geglüht wird.

Hiermit werden die Korngrenzen im metallischen Band entspannt und die Korngrößen gleichmäßiger, so dass die Gefahr einer Überbeanspruchung, insbesondere die Gefahr von Rissen, beim Umformen, insbesondere beim Tiefziehen, reduziert wird.

Gemäß einer bevorzugten Ausführung des erfindungsgemäßen Verfahrens kann vorgesehen sein, dass das metallische Band durch Walzen hergestellt wird, insbesondere durch Walzen mit anschließendem Glühen.

Mit diesem Verfahren kann das metallische Band auf einfache Weise kostengünstig aus einem Draht oder aus einem kompakten Stück gefertigt werden.

Des Weiteren kann vorgesehen sein, dass das metallische Band vor dem Einstanzen der sich wiederholenden Strukturen eine Dicke von 50 µm bis 2 mm aufweist, bevorzugt eine Dicke von 100 µm bis 1 mm, ganz bevorzugt eine Dicke von 100 µm bis 200 µm.

Mit diesen Dicken lassen sich die Hülsen für elektrophysiologische und neuromedizinische Anwendungen in passenden Größen fertigen. Eine Nacharbeitung der Hülsen durch Abdrehen ist dann nicht mehr notwendig.

Es wird ferner vorgeschlagen, dass die Flächen der sich wiederholenden Strukturen eine Fläche zwischen 10 mm² und 2000 mm² aufweist, bevorzugt eine Fläche zwischen 50 mm² und 1000 mm² aufweist.

Auch diese Größen dienen dazu, dass aus dem durch Stanzen strukturierten Band Hülsen mit einer für elektrophysiologische und neuromedizinische Anwendungen geeigneten Größe hergestellt werden können, so dass bei der Verarbeitung nur wenige Abfälle entstehen, die anschließend zur Wiederverwertung wieder aufgeschmolzen werden müssten.

Des Weiteren kann vorgesehen sein, dass die fertige Hülse eine Wandstärke zwischen 30 µm und 300 µm aufweist.

Diese Wandstärken sind für Hülsen für elektrophysiologische und neuromedizinische Anwendungen besonders geeignet.

Es ist für die spätere Anwendung der mit dem erfindungsgemäßen Verfahren hergestellten Hülsen besonders vorteilhaft, wenn vorgesehen ist, dass zumindest alle nach außen weisenden Strukturen der Hülse mit einem Krümmungsradius von größer als 0,1 mm hergestellt werden, bevorzugt alle Strukturen der Hülse (1) mit einem Krümmungsradius von größer als 0,1 mm hergestellt werden.

Hiermit wird erreicht, dass keine scharfen Kanten in den Bereichen vorkommen, die später mit den oft sehr feinen Drähten in Verbindung kommen. Hierdurch kann erreicht werden, dass eine Beschädigung der Drähte verhindert wird oder zumindest die Gefahr für eine Beschädigung reduziert wird. Eine Beschädigung der Drähte kann die Funktion und die Haltbarkeit des mit der Hülse aufgebauten Produkts im elektrophysiologischen Bereich beeinträchtigen.

Es wird ferner vorgeschlagen, dass der erste Bereich mit dem größeren Durchmesser eine Länge zwischen 1 mm und 20 mm aufweist, bevorzugt zwischen 4 mm und 15 mm, und/oder zweite Bereich mit dem kleineren Durchmesser eine Länge zwischen 1 mm und 10 mm aufweist, bevorzugt zwischen 2 mm und 8 mm.

Hiermit wird eine außenliegende Anschlussfläche für einen Sensor mit dem Außenumfang des ersten Bereichs bereitgestellt und mit dem Außenumfang des zweiten Bereichs eine Anschlussfläche und eine Führung für eine elektrische Kontaktierung durch einen Draht. Damit ist die hergestellte Hülse gut zur Herstellung von Kontaktierungen für Herzschrittmacher geeignet.

Gemäß einer bevorzugten Ausführungsform des erfindungsgemäßen Verfahrens kann vorgesehen sein, dass der Außendurchmesser des ersten Bereichs mit dem größeren Durchmesser zwischen 0,5 mm und 5 mm beträgt, bevorzugt zwischen 1 mm und 2,5 mm beträgt, und/oder der Außendurchmesser des zweiten Bereichs mit dem kleineren Durchmesser zwischen 0,4 mm und 4,5 mm beträgt, bevorzugt zwischen 0,5 mm und 2 mm beträgt.

Diese Maße sind für Hülsen für Schrittmacher besonders gut geeignet.

Eine speziell bevorzugte Ausführung des erfindungsgemäßen Verfahrens kann vorsehen, dass im zweiten Bereich mit dem kleineren Durchmesser ein umlaufender Flansch zum Anschweißen einer Spule oder eines Drahts ausgeformt wird, wobei der Außendurchmesser des Flanschs zumindest um 0,1 mm größer ist als der Außendurchmesser des zweiten Bereichs, bevorzugt zwischen 0,5 mm und 5 mm größer ist als der Außendurchmesser des zweiten Bereichs.

Hiermit wird eine Hülse bereitgestellt, an die ein Draht zur elektrischen Kontaktierung der Hülse an einer definierten Stelle, nämlich dem Flansch, angebracht werden kann. Der Flansch kann zudem als Stütze für eine Federung des Drahts verwendet werden, wenn der Draht umlaufend als Feder um den zweiten Bereich herum gewickelt wird. Des Weiteren kann der Flansch auch als Begrenzung dienen, die ein weiteres Einschieben der Hülse in einen Schlauch, insbesondere einen Kunststoffschlauch, verhindert.

Es kann auch vorgesehen sein, dass am Außenumfang der Hülse des ersten und/oder des zweiten Bereichs Kontaktierungsstrukturen, insbesondere Vertiefungen und/oder Ausnehmungen, vorgesehen sind, die zum Verbindung von anderen Komponenten durch Schweißen, Crimpen, Löten und/oder Kleben geeignet sind.

Hierdurch kann die Hülse leichter mit weiteren Komponenten, wie beispielsweise Sensoren, Drähten, Schläuchen oder elektronischen Bauteilen, verbunden werden.

Des Weiteren kann vorgesehen sein, dass bei den Umformschritten die Hülsen-Form derart umgeformt wird, dass die Hülse neben dem ersten rohrförmigen Bereich mit dem größeren Durchmesser und dem zweiten rohrförmigen Bereich mit dem kleineren Durchmesser einen dritten rohrförmigen Bereich aufweist, wobei der dritte Bereich einen kleineren Durchmesser aufweist als der erste rohrförmigen Bereich mit dem größeren Durchmesser, wobei alle drei Bereiche einteilig miteinander verbunden sind, insbesondere derart einteilig miteinander verbunden sind, dass die Hülse ein durchgehendes Röhrchen bildet.

Hiermit wird eine weitere Variante einer Hülse erzeugt, bei der in der Mitte eine Verdickung entsteht. Es kann neben dem ersten Bereich und dem zweiten Bereich also erfindungsgemäß auch noch ein dritter Bereich vorgesehen sein. Bevorzugt ist bei dieser Variante keine geschlossene Deckfläche vorgesehen.

Die Aufgaben der vorliegenden Erfindung werden auch gelöst durch ein Verfahren zum Herstellen eines neuromedizinischen Sensors oder einer elektrophysiologischen Ringelektrode umfassend ein solches Verfahren, bei dem an der Flanke des Übergangs vom ersten Bereich zum zweiten Bereich oder am Flansch ein erster Draht angeschweißt wird, wobei der erste Draht die Hülse im zweiten Bereich spiralförmig umläuft und sich in Richtung von dem ersten Bereich weg erstreckt.

Der neuromedizinische Sensor beziehungsweise die elektrophysiologische Ringelektrode kann beispielsweise zur Steuerung eines Herzschrittmachers oder einer Sonde für die tiefe Hirnstimulation verwendet werden. Der erste Draht kann dann gegen die Flanke oder den Flansch federnd gelagert werden. Hiermit können die Bewegungen, die im Körper stattfinden, ausgeglichen werden, ohne dass intensive mechanische Belastungen auf die Verbindung zwischen dem ersten Draht und der Hülse auftreten.

Dabei kann vorgesehen sein, dass durch das Innere der Hülse ein zweiter Draht gezogen wird, insbesondere ein spiralförmiger zweiter Draht gezogen wird, der gegen die Hülse und den ersten Draht elektrisch isoliert ist.

Der zweite Draht kann als Sonde zur elektrischen Stimulation von Muskel- oder Nervenzellen verwendet werden. Eine mit dem Verfahren hergestellte Elektrode kann so als elektrophysiologische Elektrode eingesetzt werden.

Ferner kann vorgesehen sein, dass außen an dem ersten Bereich der Hülse eine Elektrode zur Messung einer Änderung der elektrischen Feldstärke und/oder eine Kontaktfläche für eine Elektrode zur Messung einer Änderung der elektrischen Feldstärke befestigt wird, der oder die mit dem ersten Draht elektrisch leitend verbunden ist oder sind.

Hiermit kann die Hülse als Elektrode im elektrophysiologischen Bereich verwendet werden, mit der eine muskuläre oder neurologische Reaktion gemessen werden kann. Mit der elektrophysiologischen Elektrode kann dann auch ein Sensor aufgebaut werden.

Der Erfindung liegt die überraschende Erkenntnis zugrunde, dass es durch Umformung eines metallischen Bands gelingt, die Hülsen aufzubauen, ohne dass dabei große Mengen an Abfall des Materials entstehen, aus dem die Hülse gefertigt wird. Gleichzeitig können bei derartigen Umformschritten, wie Tiefzieh-Schritten, scharfe Kanten vermieden und dadurch die Gefahr einer Beschädigung der Drähte zum Kontaktieren der mit der Hülse hergestellten Ringelektrode reduziert werden. Das erfindungsgemäße Verfahren lässt sich schnell durchführen und ist kostengünstig umsetzbar. Das erfindungsgemäße Verfahren ist auch leicht variierbar, insbesondere dann, wenn ein Werkzeug verwendet wird, in dem mehrere austauschbare Module enthalten sind, mit denen in einzelnen Umformschritten, insbesondere Tiefzieh-Schritten, Stanzschritten oder Stanzumformschritten, eine schrittweise weiterführende Umformung des Bands zur Hülse erfolgt. Aufgrund des notwendigen hohen Umformungsgrads wird erfindungsgemäß vorgesehen, dass die Umformung des Bands zur Hülse mit mehreren Umformschritten erfolgt, bevorzugt in wenigstens drei oder wenigstens vier Umformschritten erfolgt. Dadurch wird sichergestellt, dass die Umformung nicht zu einer Zerstörung des Materials beziehungsweise zu einer Beschädigung der erzeugten Hülse führt. Die Form der Hülse und das Material der Hülse sind für elektrophysiologische und neuromedizinische Anwendungen geeignet.

Der neue Fertigungsansatz vermeidet die Nachteile des Stands der Technik, da als Ausgangsmaterial ein dünnes Band eingesetzt werden kann und somit nur geringe Edelmetallverluste zu verzeichnen sind. Weiterhin ist dieses Verfahren sehr kostengünstig und liefert qualitativ hochwertige und reproduzierbare Ergebnisse. Das erfindungsgemäße Verfahren erlaubt eine kostengünstige Herstellung von Ringelektroden und Komponenten aus Edelmetall oder enthaltend zumindest ein Edelmetall zur Verwendung im elektrophysiologischen und neuromedizinischen Bereich, insbesondere als Stimulations- oder Messelektrode im Bereich der aktiven Implantate. Während ein Drehprozess höheren Variationen aufgrund von Werkzeugverschleiß unterliegt ist ein erfindungsgemäßes Forming wesentlich wiederholgenauer, so dass die erfindungsgemäß erzeugten Hülsen einer deutlich geringeren Variation beziehungsweise Abweichung untereinander unterliegen.

Im Folgenden werden Ausführungsbeispiele der Erfindung anhand von zehn schematisch dargestellten Figuren erläutert, ohne jedoch dabei die Erfindung zu beschränken. Dabei zeigt:
Figur 1: eine schematische perspektivische Darstellung von vier erfindungsgemäßen Hülsen, die mit einem erfindungsgemäßen Verfahren hergestellt wurden;
Figur 2: eine schematische Querschnittansicht durch eine erfindungsgemäße Hülse, die mit einem erfindungsgemäßen Verfahren hergestellt wurde;
Figur 3: eine Teilansicht der schematischen Querschnittansicht nach Figur 2, in der der kleinere Bereich der Hülse und die Abschrägung der Kanten dargestellt ist;
Figur 4: eine schematische Querschnittansicht durch eine weitere erfindungsgemäße Hülse mit drei Bereichen, die mit einem erfindungsgemäßen Verfahren hergestellt wurde;
Figur 5: eine schematische Querschnittansicht durch eine weitere erfindungsgemäße Hülse mit einer geschlossenen Deckfläche, die mit einem erfindungsgemäßen Verfahren hergestellt wurde;
Figur 6: eine schematische Querschnittansicht durch eine weitere erfindungsgemäße Hülse mit einer geschlossenen Deckfläche und Flansch, die mit einem erfindungsgemäßen Verfahren hergestellt wurde;
Figur 7: eine schematische Querschnittansicht durch eine weitere erfindungsgemäße Hülse mit einer geschlossenen Deckfläche, Flansch und Ausnehmungen, die mit einem erfindungsgemäßen Verfahren hergestellt wurde; Figur 8: eine schematische perspektivische Ansicht einer Maschine zum Durchführen eines erfindungsgemäßen Verfahrens;
Figur 9: eine schematische Querschnittansicht der Maschine nach Figur 8; und
Figur 10: eine schematische perspektivische Ansicht eines Bands während der Umformung mit einem erfindungsgemäßen Verfahren.

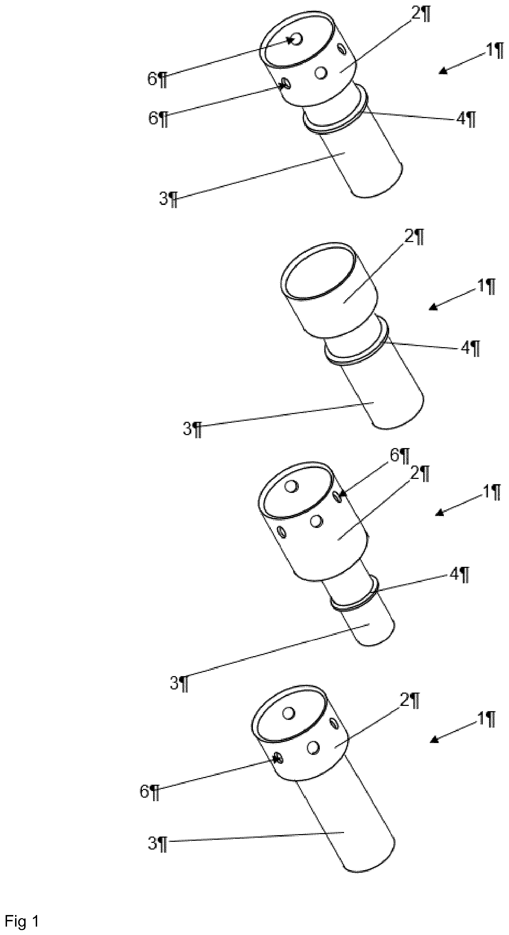

In Figur 1 sind vier verschiedene Hülsen 1 in einer schematischen perspektivischen Darstellung gezeigt, die mit erfindungsgemäßen Verfahren herstellbar sind. Jede Hülse 1 weist einen ersten Bereich 2 mit einem größeren Durchmesser und einen zweiten Bereich 3 mit einem kleineren Durchmesser auf. Die Hülsen 1 bilden durchgehende Röhrchen, wobei der Innendurchmesser im ersten Bereich 2 größer ist als der Innendurchmesser im zweiten Bereich 3. Grundsätzlich weisen die Hülsen 1 eine gleichmäßige Wandstärke zwischen 0,01 mm und 0,1 mm auf und bestehen aus einem Edelmetall, wie beispielsweise und erfindungsgemäß bevorzugt Platin, oder aus einer Edelmetallverbindung, wie beispielsweise und erfindungsgemäß bevorzugt eine Platin-Iridium-Legierung.

Im zweiten Bereich 3 kann ein Flansch 4 vorgesehen sein, der als Erhebung über den Außendurchmesser des zweiten Bereichs 3 hinaus vollständig um den zweiten Bereich 3 herum läuft. Ein solcher Flansch 4 kann beispielsweise durch eine Art Faltung bei einem Tiefzieh-Schritt oder bei zwei Tiefzieh-Schritten während eines schrittweisen Umformens aus einem flächigen metallischen Ausgangsmaterial (dem Edelmetall oder der Edelmetallverbindung) erzeugt werden. Im ersten Bereich 2 können Ausnehmungen 6 vorgesehen sein, die sich im ersten Bereich 2 ins Innere der Hülse 1 erstrecken. Zusätzlich können Vertiefungen (nicht gezeigt) eingeprägt sein. Die Vertiefungen dienen später als elastische Befestigungselemente für Schlauchmäntel und können durch Eindrücken aus Richtung der Zylindermantelwand im ersten Bereich 2 erzeugt werden. Die Ausnehmungen 6 können auch in den Vertiefungen vorgesehen sein. Die Ausnehmungen 6durchbrechen die Wandung des ersten Bereichs 2. Die Ausnehmungen 6 können durch Stanzen erzeugt werden oder die Ausnehmungen 6 werden gleichzeitig mit den Vertiefungen durch einen gemeinsamen Stanz-Tiefzieh-Schritt erzeugt, bei dem ein zum Stanzen und Tiefziehen geeignetes Modul eines Werkzeugs zum Einsatz kommt.

Bis auf die Ausnehmungen 6 und gegebenenfalls die Vertiefungen sind die Hülsen 1 rotationssymmetrisch bezüglich einer Zylinderachse aufgebaut, die in Richtung der durchgehenden Öffnung des durch die Hülse 1 gebildeten Röhrchens verläuft. An den Flansch 4 oder an die Flanke zwischen dem ersten Bereich 2 und dem zweiten Bereich 3 können zur Herstellung und Anwendung der Ringelektrode später Drähte (nicht gezeigt) angeschweißt werden, die der elektrischen Kontaktierung der Hülse 1 dienen.

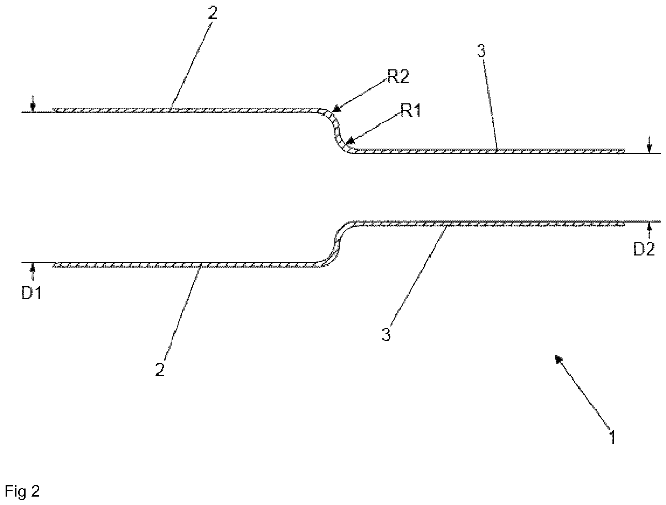

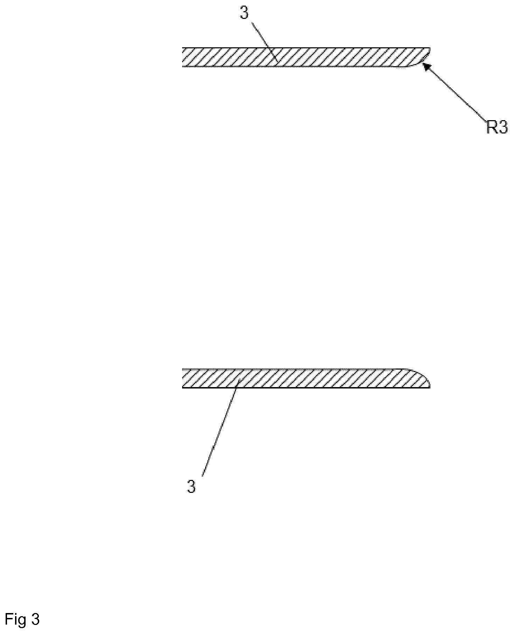

Figur 2 zeigt eine schematische Querschnittansicht durch eine erfindungsgemäße Hülse 1, die mit einem erfindungsgemäßen Verfahren hergestellt wurde, und Figur 3 eine Teilansicht der schematischen Querschnittansicht nach Figur 2, in der der kleinere zweite Bereich 3 der Hülse 1 und die Abschrägung der Kanten mit einem Radius R3 dargestellt ist. Der Durchmesser D1 der Hülse 1 ist im ersten Bereich 2 größer als der Durchmesser D2 im zweiten Bereich 3.

Aufgrund der Umformung durch Tiefziehen entstehen an den Übergängen zwischen dem ersten Bereich 2 und dem zweiten Bereich 3 keine scharfen Kanten, die zu einer Beeinträchtigung von mit der Hülse 1 zu verbindenden Drähten (nicht gezeigt) führen könnten. Stattdessen entstehen Krümmungsradien R1, R2 von wenigstens 0,1 mm oder vorzugsweise von wenigstens 0,5 mm, die aufgrund der Abrundung nicht oder nicht so leicht zu einer mechanischen Beschädigung der Drähte führen. In Figur 2 ist zudem gut zu erkennen, dass die Hülse 1 eine durchgehende Öffnung (in Figur 2 von links nach rechts) aufweist.

In gleicher Weise sind die Kanten der Hülse 1 abgerundet, wie dies besonders in Figur 3 gut zu erkennen ist. Der Krümmungsradius R3 hierzu beträgt ebenfalls zumindest 0,1 mm, so dass auch an dieser Stelle die Gefahr einer Beschädigung der mit der Hülse 1 kontaktierten Drähte reduziert ist.

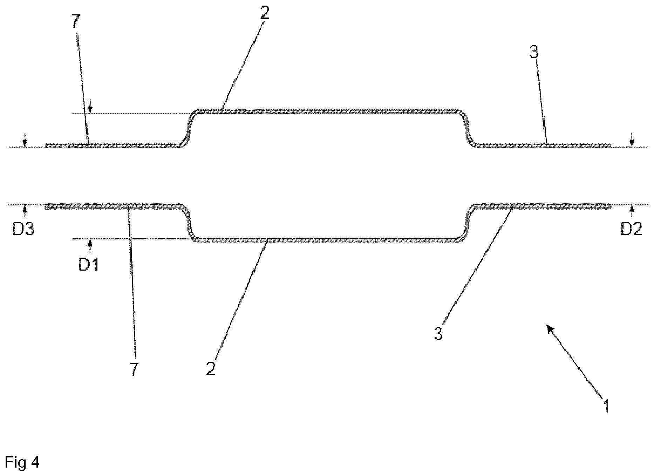

Figur 4 zeigt eine schematische Querschnittansicht durch eine weitere erfindungsgemäße Hülse 1, die mit einem erfindungsgemäßen Verfahren hergestellt wurde. Die Hülse 1 weist einen ersten Bereich 2 mit einem größeren Durchmesser und einen zweiten Bereich 3 mit einem kleineren Durchmesser sowie einen dritten Bereich 7 mit einem kleineren Durchmesser auf. Der Innendurchmesser D1 des ersten Bereichs 2 ist größer als die Innendurchmesser D2, D3 des zweiten Bereichs 3 und des dritten Bereichs 7. Der Innendurchmesser D2 des zweiten Bereichs 3 kann größer, gleich groß oder auch kleiner sein als der Innendurchmesser D3 des dritten Bereichs 7.

Die Hülse 1 bildet ein durchgehendes Röhrchen. Grundsätzlich weist die Hülse 1 eine gleichmäßige Wandstärke zwischen 0,01 mm und 0,1 mm auf und besteht aus einem Edelmetall oder aus einer intermetallischen Legierung mit einem Edelmetall als Hauptkomponente. Die Krümmungsradien an den Übergängen vom zweiten Bereich 3 zum ersten Bereich 2 sowie vom ersten Bereich 2 zum dritten Bereich 7 sind derart ausgeformt, dass sie zumindest 0,1 mm betragen. Durch die durchgehende Hülse können Drähte (nicht gezeigt) durchgeführt werden. Zudem kann die Hülse 1 selbst mit einem Draht (nicht gezeigt) elektrisch kontaktiert werden, um eine elektrophysiologische Elektrode zu bilden.

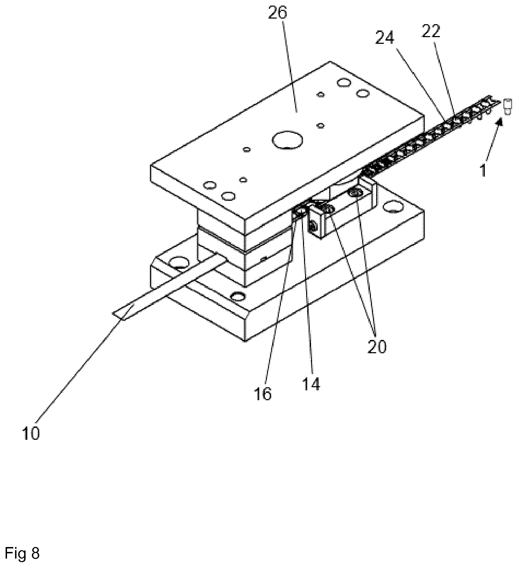

Bei der Herstellung der Hülse 1 nach Figur 4 wird zunächst der erste Bereich 2 aus einer Fläche eines Bands (siehe Figuren 8 bis 10) tiefgezogen. Anschließend wird durch Tiefziehen im ersten Bereich 2 der zweite Bereich 3 erzeugt. Diese beiden Tiefziehschritte können in mehreren separaten Umformschritten erfolgen, indem senkrecht zu der Fläche, aus der die Bereiche 2, 3 tiefgezogen werden, Stempel auf diese Fläche aufgedrückt werden. Danach kann aus dem ersten Bereich 2 durch Pressen auf die Seitenwände des ersten Bereichs 2 der dritte Bereich 7 durch Tiefziehen geformt werden. Als letzter Schritt wird die Hülsen-Form freigestanzt, so dass die Hülse 1 nach Figur 4 vorliegt.

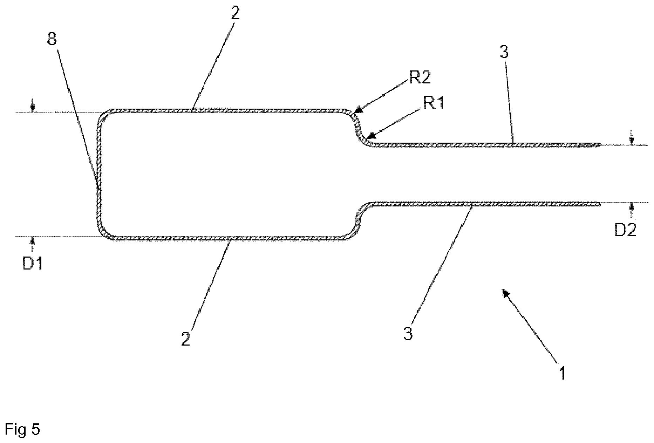

Figur 5 zeigt eine weitere schematische Querschnittansicht durch eine weitere erfindungsgemäße Hülse 1 mit einer geschlossenen Deckfläche 8, die mit einem erfindungsgemäßen Verfahren hergestellt wurde. Die Hülse 1 weist einen ersten Bereich 2 mit einem größeren Durchmesser und einen zweiten Bereich 3 mit einem kleineren Durchmesser auf. Der Innendurchmesser D1 des ersten Bereichs 2 ist größer als der Innendurchmesser D2 des zweiten Bereichs 3.

Die Hülse 1 bildet ein einseitig geschlossenes Röhrchen. Grundsätzlich weist die Hülse 1 eine gleichmäßige Wandstärke zwischen 0,01 mm und 0,1 mm auf und besteht aus Tantal, Titan, einem Edelmetall oder aus einer intermetallischen Legierung damit oder daraus. Die Krümmungsradien R1, R2 an den Übergängen vom zweiten Bereich 3 zum ersten Bereich 2 sind derart ausgeformt, dass sie zumindest 0,1 mm betragen. In die Hülse 1 kann ein Draht (nicht gezeigt) eingeführt und elektrisch kontaktiert werden, um eine elektrophysiologische Elektrode zu bilden.

Bei der Herstellung der Hülse 1 nach Figur 5 wird zunächst der erste Bereich 2 aus einer Fläche eines Bands (siehe Figuren 8 bis 10) tiefgezogen. Dieser Tiefziehschritt kann in mehreren separaten Umformschritten erfolgen, indem senkrecht zu der Fläche, aus der der erste Bereich 2 tiefgezogen wird, Stempel auf diese Fläche aufgedrückt werden. Danach kann aus dem ersten Bereich 2 durch Pressen auf die Seitenwände des ersten Bereichs 2 der zweite Bereich 3 durch Tiefziehen geformt werden. Als letzter Schritt wird die Hülsen-Form freigestanzt, so dass die Hülse 1 nach Figur 5 vorliegt. Dabei bleibt die Deckfläche 8 geschlossen und wird nicht freigestanzt.

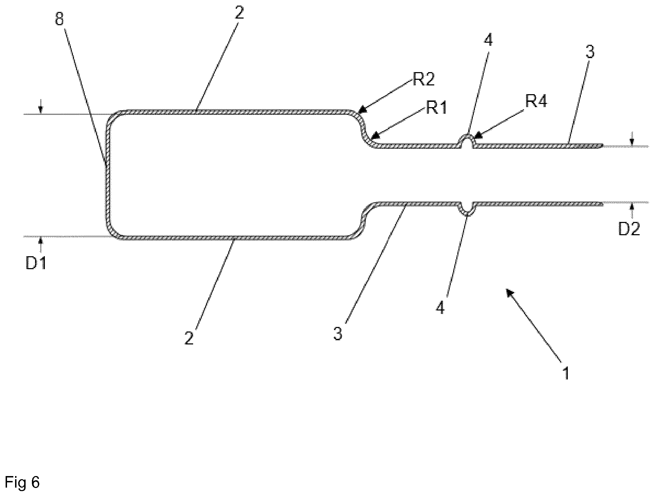

Figur 6 zeigt eine schematische Querschnittansicht durch eine weitere erfindungsgemäße Hülse 1 mit einer geschlossenen Deckfläche 8 und einem Flansch 4, die mit einem erfindungsgemäßen Verfahren hergestellt wurde. Die Hülse 1 weist einen ersten Bereich 2 mit einem größeren Durchmesser und einen zweiten Bereich 3 mit einem kleineren Durchmesser auf. Der Innendurchmesser D1 des ersten Bereichs 2 ist größer als der Innendurchmesser D2 des zweiten Bereichs 3. Im zweiten Bereich 3 der Hülse 1 ist ein umlaufender Flansch 4 ausgeformt, der sich über den Außendurchmesser des zweiten Bereichs 3 erhebt.

Die Hülse 1 bildet ein einseitig geschlossenes Röhrchen. Grundsätzlich weist die Hülse 1 eine gleichmäßige Wandstärke zwischen 0,01 mm und 0,1 mm auf und besteht aus Tantal, Titan, einem Edelmetall oder aus einer intermetallischen Legierung damit oder daraus. Die Krümmungsradien R1, R2 an den Übergängen vom zweiten Bereich 3 zum ersten Bereich 2 und die nach außen weisenden Krümmungsradien R4 des Flanschs 4 sind derart ausgeformt, dass sie zumindest 0,1 mm betragen. In die Hülse 1 kann ein Draht (nicht gezeigt) eingeführt und elektrisch kontaktiert werden, um eine elektrophysiologische Elektrode zu bilden.

Bei der Herstellung der Hülse 1 nach Figur 6 wird zunächst der erste Bereich 2 aus einer Fläche eines Bands (siehe Figuren 8 bis 10) tiefgezogen. Dieser Tiefziehschritt kann in mehreren separaten Umformschritten erfolgen, indem senkrecht zu der Fläche, aus der der erste Bereich 2 tiefgezogen wird, Stempel auf diese Fläche aufgedrückt werden. Danach kann aus dem ersten Bereich 2 durch Pressen auf die Seitenwände des ersten Bereichs 2 der zweite Bereich 3 durch Tiefziehen geformt werden. Danach oder dabei wird der Flansch 4 durch Tiefziehen in bekannter Weise erzeugt. Als letzter Schritt wird die Hülsen-Form freigestanzt, so dass die Hülse 1 nach Figur 6 vorliegt. Dabei bleibt die Deckfläche 8 geschlossen und wird nicht freigestanzt.

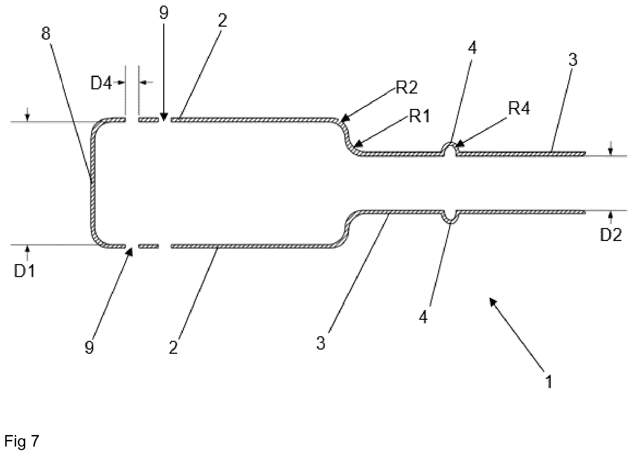

Figur 7 zeigt eine schematische Querschnittansicht durch eine weitere erfindungsgemäße Hülse 1 mit einer geschlossenen Deckfläche 8, Flansch 4 und Ausnehmungen 9, die mit einem erfindungsgemäßen Verfahren hergestellt wurde. Die Hülse 1 weist einen ersten Bereich 2 mit einem größeren Durchmesser und einen zweiten Bereich 3 mit einem kleineren Durchmesser auf. Der Innendurchmesser D1 des ersten Bereichs 2 ist größer als der Innendurchmesser D2 des zweiten Bereichs 3. Im zweiten Bereich 3 der Hülse 1 ist ein umlaufender Flansch 4 ausgeformt, der sich über den Außendurchmesser des zweiten Bereichs 3 erhebt.

Die Hülse 1 bildet ein einseitig geschlossenes Röhrchen. Grundsätzlich weist die Hülse 1 eine gleichmäßige Wandstärke zwischen 0,01 mm und 0,1 mm auf und besteht aus Tantal, Titan, einem Edelmetall oder aus einer intermetallischen Legierung damit oder daraus. Die Krümmungsradien R1, R2 an den Übergängen vom zweiten Bereich 3 zum ersten Bereich 2 und die nach außen weisenden Krümmungsradien R4 des Flanschs 4 sind derart ausgeformt, dass sie zumindest 0,1 mm betragen. In die Hülse 1 kann ein Draht (nicht gezeigt) eingeführt und elektrisch kontaktiert werden, um eine elektrophysiologische Elektrode zu bilden.

Bei der Herstellung der Hülse 1 nach Figur 6 wird zunächst der erste Bereich 2 aus einer Fläche eines Bands (siehe Figuren 8 bis 10) tiefgezogen. Dieser Tiefziehschritt kann in mehreren separaten Umformschritten erfolgen, indem senkrecht zu der Fläche, aus der der erste Bereich 2 tiefgezogen wird, Stempel auf diese Fläche aufgedrückt werden. Danach kann aus dem ersten Bereich 2 durch Pressen auf die Seitenwände des ersten Bereichs 2 der zweite Bereich 3 durch Tiefziehen geformt werden. Danach oder dabei wird der Flansch 4 durch Tiefziehen in bekannter Weise erzeugt. Als letzter Schritt wird die Hülsen-Form freigestanzt, so dass die Hülse 1 nach Figur 6 vorliegt. Dabei bleibt die Deckfläche 8 geschlossen und wird nicht freigestanzt.

Zudem sind bei der Ausführung nach Figur 7 eine Vielzahl von Ausnehmungen 9 in die Wandung des ersten Bereichs 2 eingestanzt worden. Diese können beispielsweise dazu dienen, einen Flüssigkeit durch die Hülse 1 und die Ausnehmungen 9 fließen lassen zu können. Die Flüssigkeit kann zum Kühlen einer mit der Hülse 1 hergestellten Ringelektrode verwendet werden. Die Ausnehmungen 9 können aber auch alternativ oder einige der Ausnehmungen 9 können zur Befestigung von Bauteilen, wie Drähten (nicht gezeigt) oder Isolationsschläuchen (nicht gezeigt) der elektrophysiologischen Elektrode verwendet werden.

Die Kanten der Hülsen 1 nach den Figuren 4 bis 7 sind analog zu Figur 3 ausgeführt.

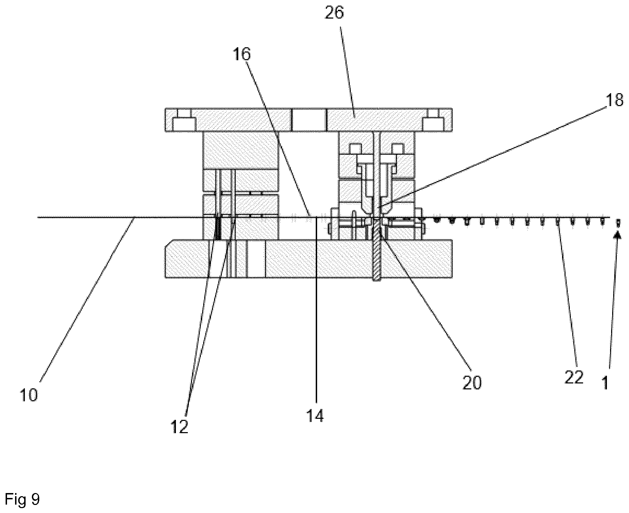

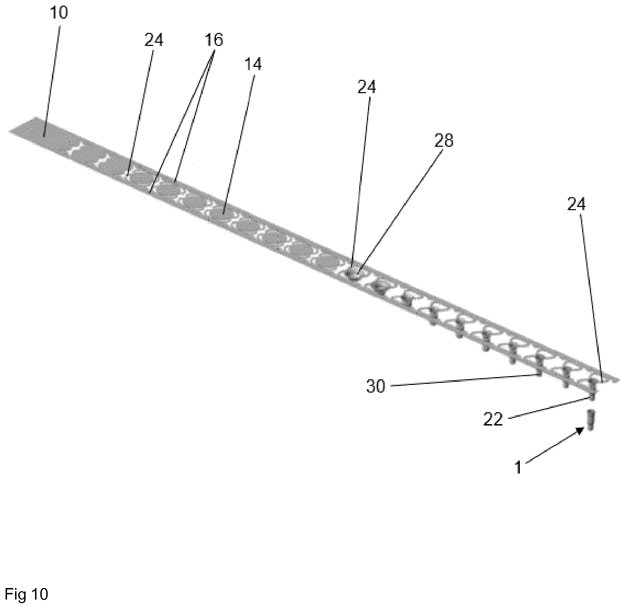

Figur 8 zeigt eine schematische perspektivische Ansicht der wesentlichen Teile einer Maschine, die zum Durchführen eines erfindungsgemäßen Verfahrens vorgesehen ist. Figur 9 zeigt hierzu eine schematische Querschnittansicht der Maschine nach Figur 8. Die Umformung des Bands 10 ist dazu separat in einer perspektivischen Darstellung des Bands 10 in Figur 10 dargestellt. In den Figuren 8 und 9 ist dadurch schematisch ein Werkzeug dargestellt, mit dem das Band 10 umzuformen ist. Das erste Werkzeugteil beziehungsweise das erste Werkzeug (links) dient dem Stanzen von Strukturen in das Band 10 und das zweite Werkzeugteil beziehungsweise das zweite Werkzeug dient der Umformung des durch das Stanzen vorgeformten Bands 10.

Ein metallisches Band 10 aus einem Edelmetall oder einer Legierung beziehungsweise Verbindung mit einem Edelmetall als Hauptkomponente wird durch die Maschine gefördert und dabei bearbeitet, beziehungsweise das Band 10 wird in mehreren Schritten umgeformt, um daraus eine Hülse 1 herzustellen. In den Figuren 8 und 9 ist das Band 10 während der Umformung mit einem erfindungsgemäßen Verfahren gezeigt, um eine bessere Übersicht über die einzelnen Schritte der Umformung des Bands 10 zur Herstellung der Hülse 1 zu geben.

In einem ersten Teil der Maschine (in Figur 8 und 9 links) wird das Band 10 mit Hilfe von mehreren Stanzen 12 bearbeitet. Dabei werden Teile des Bands 10 ausgestanzt, um sich wiederholende Strukturen im Band 10 zu erzeugen, die später zu Hülsen 1 umgeformt werden. Es können auch mehr als nur zwei Stanz-Schritte erfolgen. Theoretisch ist es auch möglich, das Stanzen der sich weiderholenden Strukturen auch nur in einem Schritt mit einem einzigen Stanzmodul des ersten Teils der Maschine zu erzeugen. Nach dem Stanzen sind in dem Band 10 sich wiederholende Strukturen (siehe auch Figur 10) ausgeformt, wobei jede sich wiederholende Struktur eine innere Fläche 14 in Form einer Kreisscheibe mit 5 bis 20 mm Durchmesser aufweist, je nach Größe der herzustellenden Hülse 1. Jede innere Fläche 14 ist über vier Stege 24 mit zwei äußeren Streifen 16 verbunden, die die inneren Flächen 14 beidseitig flankieren und das Band 10 zusammenhalten. Hierzu ist zu beachten, dass in Figur 9 aufgrund der Seitenansicht die Strukturierung des Bands 10 nur anhand von senkrechten Hilfslinien zu erkennen ist.

In einem zweiten Teil (in Figur 8 und 9 rechts) der Maschine werden mit mehreren Modulen, von denen in Figur 9 der Übersichtlichkeit wegen nur eines dargestellt ist, die inneren Flächen 14 des Bands 10 mit jeweils einem Stempel 18 und jeweils einer Pressform 20 durch Tiefziehen schrittweise umgeformt. Die einzelnen Stufen beziehungsweise Schritte der Umformung sind besonders gut in Figur 10 zu erkennen. Durch das Tiefziehen mit den Modulen in mehreren Schritten wird eine Hülsen-Form 22 erzeugt beziehungsweise weiter bearbeitet, die über die Stege 24 mit den äußeren Streifen 16 verbunden bleibt, so dass das Band 10 einfach in der Maschine befördert und prozessiert werden kann. Die beiden Teile der Maschine werden mit einer automatischen Presse 26 zusammengepresst. Zwischen den einzelnen Umformschritten wird das Band 10 so weiter transportiert, dass die sich wiederholenden Strukturen beziehungsweise die Hülsen-Formen 22 zum nächsten Modul weiter transportiert werden, also beispielsweise genau um die Länge einer sich wiederholenden Struktur des Bands 10. Die Hülsen-Form 28 (siehe Figur 10) nach dem ersten Tiefzieh-Schritt ist nur wenig umgeformt, während die Hülsen-Form 30 (siehe Figur 10) nach dem letzten Tiefzieh-Schritt bis auf den geschlossenen Boden schon fertig ausgeformt ist. Der zunächst geschlossene Boden des unteren Teils (des zweiten Bereichs 3 nach Figur 1 und 3) der Hülsen-Form 30 wird mit einer weiteren Stanze (nicht gezeigt) ab gestanzt oder mit einem Laser oder einem anderen Verfahren aufgeschnitten. Anschließend wird die fertige Hülsen-Form 22 freigestanzt, das heißt, die Stege 24 werden im Bereich der Hülsen-Form 22 aufgetrennt. Die fertige Hülse 1 kann dann als Ringelektrode eingesetzt und mit Drähten (nicht gezeigt) elektrisch kontaktiert werden.

Durch das Umformen des Bands 10 mit Tiefzieh-Schritten werden Hülsen 1 erzeugt, die einen Krümmungsradius R1, R2 von zumindest 0,1 mm aufweisen, so dass bei einem anschließenden Kontaktieren der Hülsen 1 mit Drähten (nicht gezeigt) keine Beschädigungen der Drähte durch scharfe Kanten der Hülsen 1 auftreten können. Gleichzeitig können mit Hilfe des Bands 10 und dem Werkzeug sowie den Modulen darin in kurzer Zeit viele Hülsen 1 kostengünstig hergestellt werden. Durch die variabel einzusetzenden Module und die schrittweise Umformung kann die Maschine auch ohne großen Aufwand auf andere Arten von Hülsen 1, wie sie beispielsweise in Figur 1 gezeigt sind, umgerüstet werden. Dabei können auch für unterschiedliche Hülsen 1 teilweise die gleichen Module verwendet werden und so die Kosten und der Aufwand beim Umbau reduziert werden.

Es kann vorgesehen sein, dass die Enden beziehungsweise Kanten der Hülsen 1 durch die Umformung mit einem Krümmungsradius R3 abgeschrägt werden oder geprägt werden, so dass auch an diesen Stellen scharfe Kanten vermieden werden.

### Bezugszeichenliste

- 1: Hülse
- 2: Bereich mit großem Durchmesser
- 3: Bereich mit kleinem Durchmesser
- 4: Flansch
- 6: Ausnehmung
- 7: Dritter Bereich mit kleinem Durchmesser
- 8: Geschlossene Deckfläche
- 9: Ausnehmung
- 10: Metallisches Band
- 12: Stanze
- 14: Fläche zum Ausformen der Hülse
- 16: Äußerer Streifen
- 18: Stempel
- 20: Pressform
- 22: Hülsen-Form
- 24: Steg
- 26: Presse
- 28: Hülsen-Form nach erstem Tiefzieh-Schritt der Fläche
- 30: Hülsen-Form nach letztem Tiefzieh-Schritt
- D1: Durchmesser der Hülse im Bereich mit großem Durchmesser
- D2: Durchmesser der Hülse im Bereich mit kleinem Durchmesser
- D3: Durchmesser der Hülse im dritten Bereich mit kleinem Durchmesser
- D4: Durchmesser der Ausnehmung
- R1: Krümmungsradius
- R2: Krümmungsradius
- R3: Krümmungsradius
- R4: Krümmungsradius

## Patentansprüche

1. Verfahren zur Herstellung einer Hülse (1) zum Aufbau einer Ringelektrode für elektrophysiologische und neuromedizinische Anwendungen aus einem metallischen Band (10), wobei das Band (10) und die daraus gefertigte Hülse (1) aus einem biokompatiblen Metall oder einer biokompatiblen metallischen Verbindung bestehen, wobei sich wiederholende Strukturen in das Band (10) gestanzt werden, wobei die sich wiederholenden Strukturen jeweils zumindest eine Fläche (14) aufweisen, die über wenigstens einen Steg (24) mit wenigstens einem äußeren Streifen (16) verbunden sind, wobei zumindest der wenigstens eine äußere Streifen (16) die sich wiederholenden Strukturen randseitig miteinander verbindet, **dadurch gekennzeichnet, dass** anschließend mehrere der zumindest einen Flächen (14) der sich wiederholenden Strukturen in mehreren Umformschritten durch Tiefziehen in Hülsen-Form (28, 30) gebracht werden und die Hülsen-Form (28, 30) anschließend freigestanzt wird, so dass die Hülse (1) einen ersten rohrförmigen Bereich (2) mit einem größeren Durchmesser aufweist und einen zweiten rohrförmigen Bereich (3) mit einem kleineren Durchmesser aufweist, wobei der erste Bereich (2) mit dem größeren Durchmesser einen größeren Außendurchmesser und Innendurchmesser (D1) aufweist als der zweite Bereich (3) mit dem kleineren Durchmesser und die beiden Bereiche (2, 3) einteilig miteinander verbunden sind, und dass die Übergänge zwischen dem ersten Bereich (2) und dem zweiten Bereich (3) mit einem Krümmungsradius (R1, R2) von größer als 0,1 mm hergestellt werden.

2. Verfahren nach Anspruch 1, **dadurch gekennzeichnet, dass** das Band (10) und die daraus gefertigte Hülse (1) aus einem Edelmetall, aus Titan, aus Tantal oder einer metallischen Verbindung daraus oder einer metallischen Verbindung enthaltend zumindest ein Edelmetall als Hauptkomponente oder Titan oder Tantal als Hauptkomponente bestehen.

3. Verfahren nach Anspruch 1 oder 2, **dadurch gekennzeichnet, dass** alle Umformschritte, und bevorzugt auch das Stanzen der sich wiederholenden Strukturen und/oder das Freistanzen der Hülsen-Form (28, 30), mit einem einzigen Werkzeug erfolgt, bei dem das Werkzeug auf das metallische Band (10) gepresst wird, wobei in dem Werkzeug mehrere Module in einer Reihe hintereinander angeordnet sind und mit jedem Modul ein Umformschritt oder ein Freistanzschritt oder ein Stanz-Umform-Schritt durchgeführt wird.

4. Verfahren nach Anspruch 3, **dadurch gekennzeichnet, dass** nach jedem Stanzen und/oder Umformen das Band (10) zum nächsten Modul weiter transportiert wird, wobei vorzugsweise die Abstände aller Module zueinander in Förderrichtung des metallischen Bands (10) immer gleich groß sind.

5. Verfahren nach einem der vorangehenden Ansprüche, **dadurch gekennzeichnet, dass** zumindest vier, bevorzugt zumindest fünf, chronologisch separate Umformschritte erfolgen, um die Hülsen-Form (28, 30) zu erzeugen, wobei vorzugsweise das Werkzeug hierzu zumindest vier Module, besonders bevorzugt zumindest fünf Module umfasst.

6. Verfahren nach einem der vorangehenden Ansprüche, **dadurch gekennzeichnet, dass** das metallische Band (10) und die daraus gefertigten Hülsen (1) aus Tantal, Titan, Palladium, Platin, Gold oder einer Platin-Iridium-Legierung, einer Legierung aufweisend zumindest zwei der Elemente Tantal, Titan, Palladium, Platin, Gold und Iridium oder einer metallischen Verbindung mit Gold und/oder Platin als Hauptkomponente bestehen.

7. Verfahren nach einem der vorangehenden Ansprüche, **dadurch gekennzeichnet, dass** das Band (10) vor oder direkt nach dem Einstanzen der sich wiederholenden Strukturen geglüht wird, bevorzugt bei einer Temperatur zwischen 300° C und 1000° C geglüht wird, besonders bevorzugt bei einer Temperatur zwischen 500° C und 950° C geglüht wird, ganz besonders bevorzugt bei einer Temperatur zwischen 500° C und 700° C geglüht wird.

8. Verfahren nach einem der vorangehenden Ansprüche, **dadurch gekennzeichnet, dass** das metallische Band (10) durch Walzen hergestellt wird, insbesondere durch Walzen mit anschließendem Glühen.

9. Verfahren nach einem der vorangehenden Ansprüche, **dadurch gekennzeichnet, dass** das metallische Band (10) vor dem Einstanzen der sich wiederholenden Strukturen eine Dicke von 50 µm bis 2 mm aufweist, bevorzugt eine Dicke von 100 µm bis 1 mm, besonders bevorzugt eine Dicke von 100 µm bis 200 µm.

10. Verfahren nach einem der vorangehenden Ansprüche, **dadurch gekennzeichnet, dass** die Flächen (14) der sich wiederholenden Strukturen eine Fläche zwischen 10 mm² und 2000 mm² aufweist, bevorzugt eine Fläche zwischen 50 mm² und 1000 mm² aufweist.

11. Verfahren nach einem der vorangehenden Ansprüche, **dadurch gekennzeichnet, dass** die fertige Hülse (1) eine Wandstärke zwischen 30 µm und 300 µm aufweist.

12. Verfahren nach einem der vorangehenden Ansprüche, **dadurch gekennzeichnet, dass** alle Strukturen der Hülse (1) mit einem Krümmungsradius (R1, R2, R3) von größer als 0,1 mm hergestellt werden.

13. Verfahren nach einem der vorangehenden Ansprüche, **dadurch gekennzeichnet, dass** der erste Bereich (2) mit dem größeren Durchmesser eine Länge zwischen 1 mm und 20 mm aufweist, bevorzugt zwischen 4 mm und 15 mm, und/oder zweite Bereich (3) mit dem kleineren Durchmesser eine Länge zwischen 1 mm und 10 mm aufweist, bevorzugt zwischen 2 mm und 8 mm.

14. Verfahren nach einem der vorangehenden Ansprüche, **dadurch gekennzeichnet, dass** der Außendurchmesser des ersten Bereichs (2) mit dem größeren Durchmesser zwischen 0,5 mm und 5 mm beträgt, bevorzugt zwischen 1 mm und 2,5 mm beträgt, und/oder der Außendurchmesser des zweiten Bereichs (3) mit dem kleineren Durchmesser zwischen 0,4 mm und 4,5 mm beträgt, bevorzugt zwischen 0,5 mm und 2 mm beträgt.

15. Verfahren nach einem der vorangehenden Ansprüche, **dadurch gekennzeichnet, dass** im zweiten Bereich (3) mit dem kleineren Durchmesser ein umlaufender Flansch (4) zum Anschweißen einer Spule oder eines Drahts ausgeformt wird, wobei der Außendurchmesser des Flanschs (4) zumindest um 0,1 mm größer ist als der Außendurchmesser des zweiten Bereichs (3), bevorzugt zwischen 0,5 mm und 5 mm größer ist als der Außendurchmesser des zweiten Bereichs (3).

16. Verfahren nach einem der vorangehenden Ansprüche, **dadurch gekennzeichnet, dass** am Außenumfang der Hülse (1) des ersten und/oder des zweiten Bereichs (3) Kontaktierungsstrukturen (6, 9), insbesondere Vertiefungen und/oder Ausnehmungen (6, 9), vorgesehen sind, die zum Verbindung von anderen Komponenten durch Schweißen, Crimpen, Löten und/oder Kleben geeignet sind.

17. Verfahren nach einem der vorangehenden Ansprüche, **dadurch gekennzeichnet, dass** bei den Umformschritten die Hülsen-Form (28, 30) derart umgeformt wird, dass die Hülse (1) neben dem ersten rohrförmigen Bereich (2) mit dem größeren Durchmesser und dem zweiten rohrförmigen Bereich (3) mit dem kleineren Durchmesser einen dritten rohrförmigen Bereich (7) aufweist, wobei der dritte Bereich (7) einen kleineren Durchmesser aufweist als der erste rohrförmigen Bereich (2) mit dem größeren Durchmesser, wobei alle drei Bereiche (2, 3, 7) einteilig miteinander verbunden sind, insbesondere derart einteilig miteinander verbunden sind, dass die Hülse (1) ein durchgehendes Röhrchen bildet.

18. Verfahren zum Herstellen eines neuromedizinischen Sensors oder einer elektrophysiologischen Ringelektrode umfassend ein Verfahren nach einem der vorangehenden Ansprüche, **dadurch gekennzeichnet, dass** an der Flanke des Übergangs vom ersten Bereich (2) zum zweiten Bereich (3) oder am Flansch (4) ein erster Draht angeschweißt wird, wobei der erste Draht die Hülse (1) im zweiten Bereich (3) spiralförmig umläuft und sich in Richtung von dem ersten Bereich (2) weg erstreckt.

19. Verfahren nach Anspruch 18, **dadurch gekennzeichnet, dass** durch das Innere der Hülse (1) ein zweiter Draht gezogen wird, insbesondere ein spiralförmiger zweiter Draht gezogen wird, der gegen die Hülse (1) und den ersten Draht elektrisch isoliert ist.

20. Verfahren nach Anspruch 18 oder 19, **dadurch gekennzeichnet, dass** außen an dem ersten Bereich (2) der Hülse (1) ein Sensor zur Messung einer Änderung der elektrischen Feldstärke und/oder eine Kontaktfläche für einen Sensor zur Messung einer Änderung der elektrischen Feldstärke befestigt wird, der oder die mit dem ersten Draht elektrisch leitend verbunden ist oder sind.

## Claims

1. Method for the manufacture of a sleeve (1) for constructing a ring electrode for electrophysiological and neuromedical applications from a metallic band (10), wherein the band (10) and the sleeve (1) produced therefrom consist of a biocompatible metal or a biocompatible metallic compound, wherein repeating structures are stamped into the band (10), wherein the repeating structures in each case have at least one surface (14), which are connected via at least one web (24) to at least one outer strip (16), wherein at least the at least one outer strip (16) connects the repeating structures to one another at the edge, **characterized in that** several of the at least one surfaces (14) of the repeating structures are subsequently brought into sleeve shape (28, 30) in several reshaping steps by deep drawing, and the sleeve shape (28, 30) is subsequently stamped out so that the sleeve (1) has a first tubular region (2) with a larger diameter and a second tubular region (3) with a smaller diameter, wherein the first region (2) with the larger diameter has a larger outer diameter and inner diameter (D1) than the second region (3) with the smaller diameter, and the two regions (2, 3) are integrally connected to one another, **and in that** the transitions between the first region (2) and the second region (3) are manufactured with a radius of curvature (R1, R2) greater than 0.1 mm.

2. Method according to claim 1, **characterized in that** the band (10) and the sleeve (1) produced therefrom consist of a precious metal, of titanium, of tantalum or a metallic compound thereof, or a metallic compound containing at least one precious metal as the main component or titanium or tantalum as the main component.

3. Method according to claim 1 or 2, **characterized in that** all reshaping steps, and preferably also the stamping of the repeating structures and/or the stamping-out of the sleeve shape (28, 30), take place with a single die, **in that** the die is pressed onto the metallic band (10), wherein several modules are arranged in a row, one behind the other, in the die, and a reshaping step or a stamping-out step or a stamping-reshaping step is carried out with each module.

4. Method according to claim 3, **characterized in that,** after each stamping and/or reshaping, the band (10) is transported further to the next module, wherein the distances of all modules from one another in the conveying direction of the metallic band (10) are preferably always equal.

5. Method according to one of the preceding claims, **characterized in that** at least four, and preferably at least five, chronologically separate reshaping steps take place in order to produce the sleeve shape (28, 30), wherein the die preferably comprises at least four modules, and particularly preferably at least five modules, for this purpose.

6. Method according to one of the preceding claims, **characterized in that** the metallic band (10) and the sleeves (1) produced therefrom consist of tantalum, titanium, palladium, platinum, gold or a platinum-iridium alloy, an alloy having at least two of the elements tantalum, titanium, palladium, platinum, gold, and iridium, or a metallic compound with gold and/or platinum as the main component.

7. Method according to one of the preceding claims, **characterized in that,** before or directly after the stamping of the repeating structures, the band (10) is annealed - preferably at a temperature between 300 °C and 1,000 °C, particularly preferably at a temperature between 500 °C and 950 °C, and most particularly preferably at a temperature between 500 °C and 700 °C.

8. Method according to one of the preceding claims, **characterized in that** the metallic band (10) is manufactured by rolling, and in particular by rolling with subsequent annealing.

9. Method according to one of the preceding claims, **characterized in that,** before the stamping of the repeating structures, the metallic band (10) has a thickness of 50 µm to 2 mm - preferably a thickness of 100 µm to 1 mm, and particularly preferably a thickness of 100 µm to 200 µm.

10. Method according to one of the preceding claims, **characterized in that** the surfaces (14) of the repeating structures have an area of between 10 mm² and 2,000 mm², and preferably an area of between 50 mm² and 1,000 mm².

11. Method according to one of the preceding claims, **characterized in that** the finished sleeve (1) has a wall thickness of between 30 µm and 300 µm.

12. Method according to one of the preceding claims, **characterized in that** all structures of the sleeve (1) are manufactured with a radius of curvature (R1, R2, R3) greater than 0.1 mm.

13. Method according to one of the preceding claims, **characterized in that** the first region (2) with the larger diameter has a length of between 1 mm and 20 mm, and preferably between 4 mm and 15 mm, and/or the second region (3) with the smaller diameter has a length of between 1 mm and 10 mm, and preferably between 2 mm and 8 mm.

14. Method according to one of the preceding claims, **characterized in that** the outer diameter of the first region (2) with the larger diameter is between 0.5 mm and 5 mm, and preferably between 1 mm and 2.5 mm, and/or the outer diameter of the second region (3) with the smaller diameter is between 0.4 mm and 4.5 mm, and preferably between 0.5 mm and 2 mm.

15. Method according to one of the preceding claims, **characterized in that,** in the second region (3) with the smaller diameter, a circumferential flange (4) for welding a coil or a wire is shaped, wherein the outer diameter of the flange (4) is at least 0.1 mm greater than the outer diameter of the second region (3), and preferably between 0.5 mm and 5 mm greater than the outer diameter of the second region (3).

16. Method according to one of the preceding claims, **characterized in that,** on the outer circumference of the sleeve (1) of the first and/or of the second region (3), contacting structures (6, 9) - in particular, depressions and/or recesses (6, 9) - are provided, which are suitable for connecting other components by welding, crimping, soldering, and/or gluing.

17. Method according to one of the preceding claims, **characterized in that** the sleeve shape (28, 30) is reshaped during the reshaping steps in such a way that, besides the first tubular region (2) with the larger diameter and the second tubular region (3) with the smaller diameter, the sleeve (1) has a third tubular region (7), wherein the third region (7) has a smaller diameter than the first tubular region (2) with the larger diameter, wherein all three regions (2, 3, 7) are integrally connected to one another, and in particular are integrally connected to one another in such a way that the sleeve (1) forms a continuous tube.

18. Method for the manufacture of a neuromedical sensor or an electrophysiological ring electrode, comprising a method according to one of the preceding claims, **characterized in that,** onto the flank of the transition from the first region (2) to the second region (3) or onto the flange (4), a first wire is welded, wherein the first wire helically surrounds the sleeve (1) in the second region (3) and extends in the direction away from the first region (2).

19. Method according to claim 18, **characterized in that,** through the interior of the sleeve (1), a second wire is drawn - in particular, a helical second wire - which is electrically insulated from the sleeve (1) and the first wire.

20. Method according to claim 18 or 19, **characterized in that,** outside on the first region (2) of the sleeve (1), a sensor for measuring a change in the electrical field strength and/or a contact surface for a sensor for measuring a change in the electrical field strength is attached, which sensor or contact surface is or are electrically conductively connected to the first wire.

## Revendications

1. Procédé de fabrication d'un manchon (1) pour la construction d'une électrode annulaire pour applications électrophysiologiques et neuromédicales à partir d'un ruban (10) métallique, le ruban (10) et le manchon (1) fabriqué à partir de celui-ci étant constitués d'un métal biocompatible ou d'un composé métallique biocompatible, des structures répétitives étant estampées dans le ruban (10), les structures répétitives présentant respectivement au moins une surface (14), qui est reliée à au moins une bande extérieure (16) par le biais d'au moins une entretoise (24), au moins ladite au moins une bande extérieure (16) reliant les structures répétitives entre elles au niveau du bord, **caractérisé en ce que** plusieurs desdites au moins une surfaces (14) des structures répétitives sont ensuite mises en forme de manchon (28, 30) par emboutissage profond dans plusieurs étapes de façonnage et la forme de manchon (28, 30) est ensuite retirée par estampage, de telle sorte que le manchon (1) présente une première zone (2) tubulaire présentant un diamètre supérieur et une deuxième zone (3) tubulaire présentant un diamètre inférieur, la première zone (2) présentant le diamètre supérieur présentant un diamètre extérieur et un diamètre intérieur (D1) supérieurs à ceux de la deuxième zone (3) présentant le diamètre inférieur et les deux zones (2, 3) étant reliées l'une à l'autre d'un seul tenant **et en ce que** les jonctions entre la première zone (2) et la deuxième zone (3) sont fabriquées avec un rayon de courbure (R1, R2) supérieur à 0,1 mm.

2. Procédé selon la revendication 1, **caractérisé en ce que** le ruban (10) et le manchon (1) fabriqué à partir de celui-ci sont constitués d'un métal noble, de titane, de tantale ou d'un composé métallique de ceux-ci ou d'un composé métallique contenant au moins un métal noble comme composant principal ou du titane ou du tantale comme composant principal.

3. Procédé selon la revendication 1 ou 2, **caractérisé en ce que** toutes les étapes de façonnage et de préférence également l'estampage des structures répétitives et/ou le retrait par estampage de la forme de manchon (28, 30) sont effectués avec un unique outil, l'outil étant pressé sur le ruban (10) métallique, plusieurs modules étant disposés en une rangée les uns derrière les autres dans l'outil et une étape de façonnage ou une étape de retrait par estampage ou une étape de façonnage par estampage étant effectuée avec chaque module.

4. Procédé selon la revendication 3, **caractérisé en ce que,** après chaque estampage et/ou façonnage, le ruban (10) continue à être transporté jusqu'au prochain module, les écarts entre tous les modules étant de préférence toujours égaux dans le sens de transport du ruban (10) métallique.

5. Procédé selon l'une quelconque des revendications précédentes, **caractérisé en ce qu'au** moins quatre, de préférence au moins cinq, étapes de façonnage chronologiquement séparées sont effectuées pour générer la forme de manchon (28, 30), l'outil comprenant de préférence à cet effet quatre modules, de manière particulièrement préférée au moins cinq modules.

6. Procédé selon l'une quelconque des revendications précédentes, **caractérisé en ce que** le ruban (10) métallique et les manchons (1) fabriqués à partir de celui-ci sont constitués de tantale, de titane, de palladium, de platine, d'or ou d'un alliage de platine et d'iridium, d'un alliage comportant au moins deux des éléments tantale, titane, palladium, platine, or et iridium ou d'un composé métallique présentant de l'or et/ou du platine comme composant principal.

7. Procédé selon l'une quelconque des revendications précédentes, **caractérisé en ce que** le ruban (10) est recuit avant ou directement après l'estampage des structures répétitives, de préférence recuit à une température entre 300 °C et 1000 °C, de manière particulièrement préférée recuit à une température entre 500 °C et 950 °C, idéalement recuit à une température entre 500 °C et 700 °C.

8. Procédé selon l'une quelconque des revendications précédentes, **caractérisé en ce que** le ruban (10) métallique est fabriqué par laminage, en particulier par laminage suivi d'un recuit.

9. Procédé selon l'une quelconque des revendications précédentes, **caractérisé en ce que** le ruban (10) métallique présente, avant l'estampage des structures répétitives, une épaisseur de 50 µm à 2 mm, de préférence une épaisseur de 100 µm à 1 mm, de manière particulièrement préférée une épaisseur de 100 µm à 200 µm.

10. Procédé selon l'une quelconque des revendications précédentes, **caractérisé en ce que** les surfaces (14) des structures répétitives présentent une surface entre 10 mm² et 2000 mm², de préférence une surface entre 50 mm² et 1000 mm².

11. Procédé selon l'une quelconque des revendications précédentes, **caractérisé en ce que** le manchon (1) fini présente une épaisseur de paroi entre 30 µm et 300 µm.

12. Procédé selon l'une quelconque des revendications précédentes, **caractérisé en ce que** toutes les structures du manchon (1) sont fabriquées avec un rayon de courbure (R1, R2, R3) supérieur à 0,1 mm.

13. Procédé selon l'une quelconque des revendications précédentes, **caractérisé en ce que** la première zone (2) présentant le diamètre supérieur présente une longueur entre 1 mm et 20 mm, de préférence entre 4 mm et 15 mm et/ou la deuxième zone (3) présentant le diamètre inférieur présente une longueur entre 1 mm et 10 mm, de préférence entre 2 mm et 8 mm.

14. Procédé selon l'une quelconque des revendications précédentes, **caractérisé en ce que** le diamètre extérieur de la première zone (2) présentant le diamètre supérieur est situé entre 0,5 mm et 5 mm, de préférence entre 1 mm et 2,5 mm et/ou le diamètre extérieur de la deuxième zone (3) présentant le diamètre inférieur est situé entre 0,4 mm et 4,5 mm, de préférence entre 0,5 mm et 2 mm.

15. Procédé selon l'une quelconque des revendications précédentes, **caractérisé en ce que,** dans la deuxième zone (3) présentant le diamètre inférieur, une collerette (4) périphérique est formée pour le soudage d'une bobine ou d'un fil métallique, le diamètre extérieur de la collerette (4) étant au moins 0,1 mm supérieur au diamètre extérieur de la deuxième zone (3), de préférence entre 0,5 mm et 5 mm supérieur au diamètre extérieur de la deuxième zone (3).

16. Procédé selon l'une quelconque des revendications précédentes, **caractérisé en ce que** des structures de mise en contact (6, 9), en particulier des creux et/ou des évidements (6, 9), sont prévues sur la circonférence extérieure du manchon (1) de la première et/ou de la deuxième zone (3), lesquelles sont appropriées pour une liaison d'autres composants par soudage, sertissage, brasage et/ou collage.

17. Procédé selon l'une quelconque des revendications précédentes, **caractérisé en ce que,** lors des étapes de façonnage, la forme de manchon (28, 30) est façonnée de telle manière que le manchon (1) présente, en plus de la première zone (2) tubulaire présentant le diamètre supérieur et de la deuxième zone (3) tubulaire présentant le diamètre inférieur, une troisième zone (7) tubulaire, la troisième zone (7) présentant un diamètre inférieur à celui de la première zone (2) tubulaire présentant le diamètre supérieur, les trois zones (2, 3, 7) étant reliées entre elles d'un seul tenant, en particulier reliées entre elles d'un seul tenant de telle manière que le manchon (1) forme un petit tube continu.

18. Procédé de fabrication d'un capteur neuromédical ou d'une électrode annulaire électrophysiologique comprenant un procédé selon l'une quelconque des revendications précédentes, **caractérisé en ce qu'**un premier fil métallique est soudé sur le flanc de la jonction entre la première zone (2) et la deuxième zone (3) ou sur la collerette (4), le premier fil métallique entourant le manchon (1) en forme de spirale dans la deuxième zone (3) et s'étendant dans la direction s'éloignant de la première zone (2).

19. Procédé selon la revendication 18, **caractérisé en ce qu'un** deuxième fil métallique est tiré à travers l'intérieur du manchon (1), en particulier un deuxième fil métallique en forme de spirale, qui est isolé électriquement par rapport au manchon (1) et au premier fil métallique.

20. Procédé selon la revendication 18 ou 19, **caractérisé en ce qu'un** capteur pour la mesure d'une modification de l'intensité du champ électrique et/ou une surface de contact pour un capteur pour la mesure d'une modification de l'intensité du champ électrique est/sont fixé(e)(s) sur l'extérieur de la première zone (2) du manchon (1), lequel et/ou laquelle est/sont raccordé(e)(s) de manière électriquement conductrice au premier fil métallique.
